# EUROPÄISCHE PATENTSCHRIFT

(11) **EP 2 039 984 B1**
(45) Veröffentlichungstag und Bekanntmachung des Hinweises auf die Patenterteilung: **14.11.2012**
(21) Anmeldenummer: 08016794.3
(22) Anmeldetag: 24.09.2008
(51) Int. Cl.: F21S 8/00, F21V 14/00, F21V 33/00, A61G 10/00, A61N 5/00, F21W 131/208

(54) **Beleuchtungsvorrichtung für eine medizinische Versorgungseinheit**
Lighting device for a medical supply unit
Dispositif d'éclairage pour une unité d'entretien médicale

(30) Priorität: 24.09.2007 DE 102007045456
(43) Veröffentlichungstag der Anmeldung: 25.03.2009
(73) Patentinhaber: TRUMPF Medizin Systeme GmbH + Co. KG, 07318 Saalfeld (DE)
(72) Erfinder: Bauer, Georg, 85221 Dachau (DE); Brunner, Jürgen, 82335 Berg (DE)
(74) Vertreter: Prüfer & Partner GbR European Patent Attorneys

(56) Entgegenhaltungen:
- CA-A- 1 008 428
- DE-A1- 3 533 229
- DE-A1- 3 714 196
- DE-A1- 4 242 258
- DE-U1- 20 105 359
- GB-A- 2 243 223
- JP-A- 63 149 971
- US-A- 3 769 502
- US-A- 4 843 782
- US-A- 5 160 193
- US-A1- 2002 198 438
- US-A1- 2004 237 202
- US-B1- 6 443 591

## Beschreibung

Die Erfindung betrifft eine Beleuchtungsvorrichtung für eine Medizinische Versorgungseinheit gemäß den Merkmalen des Oberbegriffs des Patentanspruchs 1.

Der Großteil der heutigen Beleuchtungskonzepte zur Beleuchtung von Räumen besteht hauptsächlich aus einer direkten Raumbeleuchtung und gegebenenfalls aus einer indirekten Raumbeleuchtung, die vorzugsweise an einer Raumwand oder in Deckenschienen angeordnet sein kann. Die Anordnung der direkten und/ oder der indirekten Raumbeleuchtung ist dabei nicht auf sich im Raum befindliche Gegenstände, wie beispielsweise ein Krankenbett in einem Krankenzimmer angepasst, so dass Patienten durch die Anordnung der Raumbeleuchtung geblendet werden können.

Auch ist für die Inbetriebnahme der Raumbeleuchtung eine separate Elektroinstallation und Montage erforderlich, was insbesondere bei Nachrüstungen oder Renovierungen der Raumbeleuchtung zu zusätzlichem Aufwand und damit zusätzlichen Kosten führt. Ferner weist die Blickrichtung von bettlägerigen Patienten im Krankenzimmer hauptsächlich auf eine Raumdecke. Diese kann beispielsweise unangenehm aussehende Luftauslässe aufweisen, was auf den Patienten unschön bzw. unharmonisch wirkt und somit den Heilungsprozess nicht fördert.

Die Patentschrift US 3,769,502 A offenbart eine medizinische Versorgungseinheit mit einer Lichteinheit, die eine transluzente Fläche aufweist, wobei die transluzente Fläche von einer Lichtquelle der Lichteinheit beleuchtet wird, und damit den Patientenbereich beleuchtet.

Die Offenlegungsschrift DE 35 33 229 A1 zeigt eine Versorgungseinheit für eine medizinische Pflegestation mit einem Versorgungsbalken, in dem eine Lampe angeordnet ist. Durch eins durchscheinendes Mittelstück des Versorgungsbalkens wird die Raumdecke beleuchtet.

Auch die Offenlegungsschrift DE 37 14 169 A1 offenbart einen Versorgungsbalken, der in der Intensivpflege verwendet wird. Der Versorgungsbalken weist in diesem Fall oben und unten lichtdurchlässige Platten auf, durch die Licht von einer im inneren vorsehbaren Lampe abgestrahlt werden kann.

Die der vorliegenden Erfindung zu Grunde liegende Aufgabe besteht nun darin, eine Beleuchtungsvorrichtung bereitzustellen, die leicht und kostengünstig montierbar und nachrüstbar ist und die Raumbeleuchtung verbessert.

Diese Aufgabe wird erfindungsgemäß durch die Merkmale des Patentanspruchs 1 gelöst. Vorteilhafte Ausgestaltungen der Erfindung sind in den Unteransprüchen gekennzeichnet.

Die mit der Erfindung erzielten Vorteile bestehen insbesondere darin, dass eine transluzente Darstellungsfläche eine derartige Form und Größe aufweist, dass Bilder auf der Darstellungsfläche projiziert werden können, was den Aufenthaltskomfort des Patienten im Krankenzimmer erhöht, da er in einer liegenden Position z.B. Filme anschauen kann. Ferner ist die transluzente Darstellungsfläche derart auf einer Befestigungseinheit angeordnet, dass der Patient die Raumdecke, mit eventuell unangenehm aussehenden Luftauslässen und die oberhalb befindlichen Teile der Medizinischen Versorgungseinheit nicht sieht. Der Patientenblick ist hauptsächlich auf die Darstellungsfläche gerichtet, die mittels einer ersten Lichteinheit gleichmäßig, mit unterschiedlicher Helligkeit und mit unterschiedlichen Farbtemperaturen, beleuchtet werden kann und somit eine für den Patienten entspanntere Atmosphäre schafft. Die erste Lichteinheit weist dabei mindestens eine erste Lichtquelle auf. Ferner besteht ein weiterer Vorteil darin, dass die erste Lichteinheit und die Darstellungsfläche auf der Befestigungseinheit angeordnet sind. Dementsprechend sind damit die Kosten für die Montage und Nachrüstung entsprechend geringer.

Ein weiterer Aspekt der Erfindung besteht darin, dass eine erste Aufnahmeeinheit, in der die Darstellungsfläche angeordnet ist, in eine zweite Aufnahmeeinheit leicht einsetzbar ist und dort beispielsweise mit Hilfe von Rastelementen befestigt ist. Somit ist ein einfacher Ein- und Ausbau der ersten Aufnahmeeinheit aus der zweiten Aufnahmeeinheit, um eine Reinigung, Desinfektion oder einen Austausch der ersten Aufnahmeeinheit durchzuführen, möglich. Zum ergonomischen Austausch ist die zweite Aufnahmeeinheit absenkbar.

Ferner besteht eine vorteilhafte Ausgestaltung der Erfindung darin, dass die Darstellungsfläche mit Lichtstrahlen bestrahlt wird, die mit der Darstellungsfläche einen spitzen Winkel, vorzugsweise 10°, einschließen. Infolge der Bestrahlung der Darstellungsfläche unter einem spitzen Winkel wird die Darstellungsfläche gleichmäßig ausgeleuchtet, was wiederum eine entspanntere Atmosphäre für den Patienten schafft.

In vorteilhafter Weiterbildung der Erfindung ist vorgesehen, dass mittels weiterer Lichteinheiten, die eine zweite, dritte und vierte Lichtquelle aufweisen, von denen eine auf den Raumboden, eine auf die Raumdecke und eine auf die Raumseitenwände gerichtet ist, eine gesamte Ausleuchtung des Raums erzielt wird. Ferner kann damit die Raumbeleuchtung hinsichtlich Farbtemperatur, Helligkeit und Verteilung der Farbtemperatur und Helligkeit variiert werden.

Auch können die Farbtemperaturen der ersten und zweiten Lichteinheit derart aufeinander abgestimmt werden, so dass der gesamte Raum inklusive Darstellungsfläche, beispielsweise in der gleichen Farbtemperatur beleuchtet wird, und somit eine angenehmere Atmosphäre für den Patienten geschaffen wird. Ferner können die Lichtstrahlen der jeweiligen Lichtquelle der zweiten Lichteinheit derart auf die Raumseitenwände, die Raumdecke und den Raumboden gerichtet sein, dass sie mit den Raumseitenwänden, der Raumdecke und des Raumbodens einen spitzen Winkel, vorzugsweise 10°, einschließen. Somit wird eine gleichmäßigere Raumlichtverteilung ermöglicht.

Ferner besteht eine vorteilhafte Ausgestaltung der Erfindung darin, dass mittels einer dritten Lichtquelle unterschiedliche Positionen des Patientenbetts beleuchtbar sind, ohne den Patienten zu blenden. Die gewünschte Beleuchtungsposition lässt sich dabei auch durch den Patienten selbst einstellen.

Auch ist in vorteilhafter Weiterbildung der Erfindung vorgesehen, dass die Beleuchtungsvorrichtung eine Schnittstelle zum Ansteuern der jeweiligen Lichteinheit mittels eines Steuergeräts ermöglicht. Somit können mittels des Steuergeräts die Lichtstrahlen hinsichtlich der Helligkeit und seines Spektrums zur Unterstützung von Schlaf- und Wachphasen verändert werden. So hat es sich beispielsweise als vorteilhaft erwiesen, dass ein Ausschalten von blauen Licht schlaffördernd wirkt, da dadurch die Melatoninproduktion gefördert wird. Ferner besteht die Möglichkeit in dem Steuergerät feste Beleuchtungszyklen, abzuspeichern, womit die Möglichkeit geboten wird, eine für den Patienten angenehme Umgebung zu schaffen.

Auch ist in einer vorteilhaften Weiterbildung der Erfindung vorgesehen, dass an die Beleuchtungsvorrichtung ein Lichtsensor angeordnet ist. Mit Hilfe des Lichtsensors wird der Lichteinfall von natürlichem Licht, wie z. B. der Lichteinfall durch ein Fenster gemessen. Dadurch können die Lichteinheiten derart gesteuert werden, dass eine konstante Beleuchtungssituation, unabhängig vom natürlichen Lichteinfall, erzielt wird.

Ferner besteht eine vorteilhafte Ausgestaltung der Erfindung darin, dass an der Beleuchtungsvorrichtung eine Kamera angeordnet ist. Die Kamera kann zur Kommunikation eingesetzt werden, oder ergänzt eine persönliche Visite eines Arztes durch eine, abhängig vom Einzelfall auch unter Umständen nötige durchgehende Bildvisite, womit sich die Pflegesituation der Patienten verbessert und gleichzeitig auch Kosten eingespart werden können. Auch zur Videotelefonie kann die Kamera in Kombination mit der Darstellungsfläche eingesetzt werden.

Einzelheiten der Erfindung werden anhand der Zeichnung näher erläutert. Dabei zeigt:
Figur 1: eine Prinzipskizze der Beleuchtungsvorrichtung mit eingezeichneten Lichtstrahlen der jeweiligen Lichtquelle,
Figur 2: eine Prinzipskizze der Beleuchtung der Darstellungseinheit, und deren Anordnung auf einer Befestigungseinheit,
Figur 3: eine schematische Ansicht der Patientenbeleuchtung.

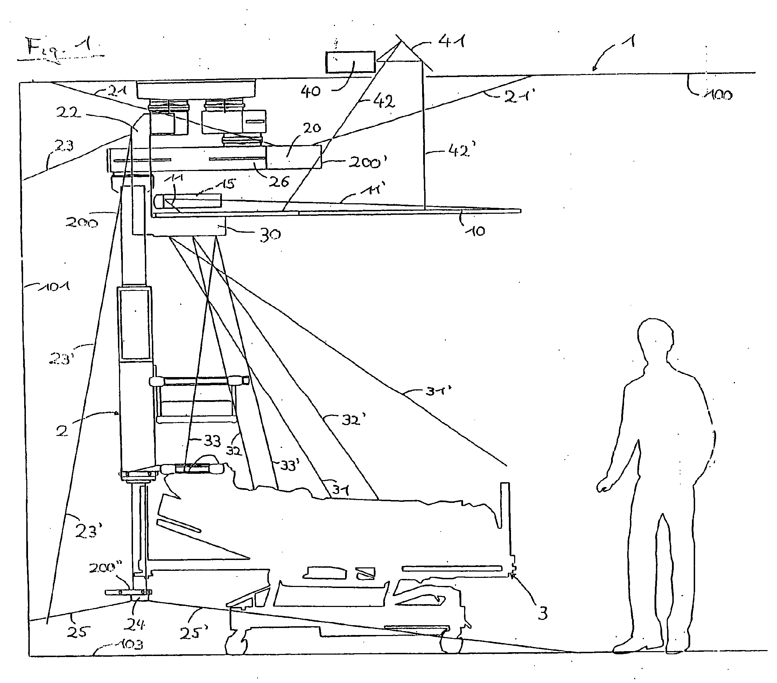

Figur 1 zeigt eine Prinzipskizze der Beleuchtungsvorrichtung mit eingezeichneten Lichtstrahlen der jeweiligen Lichtquelle. Eine Beleuchtungsvorrichtung besteht aus mehreren Lichtquellen, die über Beleuchtungseinheiten an einer Medizinischen Versorgungseinheit befestigt sind. Die Medizinische Versorgungseinheit 2 ist innerhalb eines Raums 1 angeordnet und weist Befestigungseinheiten 200, 200', 200" auf. Ferner ist innerhalb des Raums 1 ein Patientenbett 3 angeordnet. Eine Raumdecke 100 weist eine Aussparung auf, über die ein Lichtstrahl 42, 42' eines bildgebenden Geräts, wie beispielsweise eines Beamers 40, in den Raum 1 eintreten kann. Der Beamer 40 ist dabei oberhalb der Raumdecke 100 parallel zu dieser angeordnet. Die vom Beamer 40 ausgehenden Lichtstrahlen 42, 42' werden, damit sie durch die Aussparung in den Raum 1 eintreten können, mittels eines Spiegels 41 umgelenkt.

Ferner sind die Befestigungseinheiten 200, 200', 200" an der Medizinischen Versorgungseinheit 2 befestigt, die wiederum an der Decke 100 befestigt ist und weisen mehrere Lichteinheiten auf, mittels derer der Raum 1 beleuchtet wird. An der Befestigungseinheit 200 ist somit eine erste Lichteinheit angeordnet. Die erste Lichteinheit weist mindestens eine erste Lichtquelle auf, deren Lichtstrahlen 11, 11' eine in der Figur 1 nicht sichtbare Darstellungsfläche einer Darstellungseinheit 10 beleuchten. Die erste Lichteinheit ist dabei derart auf der Befestigungseinheit 200 angeordnet, dass die Lichtstrahlen 11, 11' mit der Darstellungsfläche der Darstellungseinheit 10 einen spitzen Winkel, vorzugsweise 10°, ausbilden. Ferner werden die vom Beamer 40 ausgesandten Lichtstrahlen 42, 42' vom Spiegel 41 derart umgelenkt, dass sie auf die in der Figur 1 nicht sichtbare Darstellungsfläche der Darstellungseinheit 10 treffen, und dort das gewünschte Bild erzeugt wird.

Die Darstellungseinheit 10 ist dabei parallel zur Raumdecke 100 auf der Befestigungseinheit 200 angeordnet. Dabei weist die Darstellungseinheit 10 eine derartige Länge und Breite auf, dass das durch den Beamer erzeugte Bild auf einer auf der Darstellungseinheit 10 angeordneten Darstellungsfläche projiziert werden kann, und ein möglichst großer Teil der Blickfläche des Patienten abgedeckt wird. Ferner ist die Darstellungseinheit 10 derart zwischen Raumdecke 100 und Patientenbett 3 angeordnet, dass das durch den Beamer 40 mit den ausgesandten Lichtstrahlen 42, 42' erzeugte Bild möglichst deutlich auf der Darstellungsfläche dargestellt wird. Gleichzeitig soll die Darstellungseinheit 10 einen möglichst großen Teil der Raumdecke 100 verdecken.

Ferner ist auf der Befestigungseinheit 200, 200', 200" eine zweite Lichteinheit angeordnet, die zur Beleuchtung des Raums 1 dient. Die zweite Lichteinheit weist mindestens eine, auf der Befestigungseinheit 200' angeordnete, zur Raumdecke 100 gerichtete zweite Lichtquelle 20 auf. Die Befestigungseinheit 200 weist ferner mindestens eine zu den Raumseitenwänden 101 gerichtete dritte Lichtquelle 22, und die Befestigungseinheit 200" mindestens eine zum Raumboden gerichtete vierte Lichtquelle 24 auf. Die zweite Lichtquelle 20 befindet sich dabei auf einem quer zur Befestigungseinheit 200 angeordneten Arm 26, um somit einen möglichst großen Teil der Decke 100 mit von der zweiten Lichtquelle ausgehenden Lichtstrahlen 21, 21' zu beleuchten.

Die dritte Lichtquelle 22 ist dabei an dem zur Raumdecke 100 gerichteten Ende der Befestigungseinheit 200 angeordnet und beleuchtet die Raumseitenwände 101 mit den Lichtstrahlen 23, 23'. An der Befestigungseinheit 200" ist die vierte Lichtquelle 24 angeordnet, die über die Lichtstrahlen 25, 25' den Raumboden beleuchtet. Die dritte und vierte Lichtquelle 22, 24 können dabei jeweils auf der Befestigungseinheit 200, 200" und/ oder die zweite Lichtquelle 20 kann auf dem Arm 26 derart angeordnet sein und/ oder jeweils optische Mittel aufweisen, damit die jeweiligen Lichtstrahlen der zweiten, dritten und vierten Lichtquelle 20, 22, 24 mit der Raumdecke 100, der Raumseitenwände 101 und des Raumbodens 103 einen spitzen Winkel, vorzugsweise 10°, einnehmen.

Ferner weist die Befestigungseinheit 200 eine dritte Lichteinheit 30 zur Patientenbeleuchtung auf. Die Lichteinheit 30 weist mindestens eine fünfte Lichtquelle auf, mittels derer sich mehrere Bereiche des Patientenbettes 3 unabhängig voneinander beleuchten lassen, ohne bei Ausleuchtung des Mittel- und Fußbereichs den Patienten zu blenden. So können in diesem Ausführungsbeispiel drei Bereiche des Patientenbettes beleuchtet werden. Der Kopfbereich wird mittels den Lichtstrahlen 33, 33', der Mittelbereich wird mittels den Lichtstrahlen 32, 32' und der Fußbereich mittels den Lichtstrahlen 31, 31' beleuchtet.

Die Steuerung der einzelnen Lichteinheiten kann durch den Patienten selbst oder durch eine mit einer an den Befestigungseinheiten 200, 200', 200" vorhandene Schnittstelle mittels eines externen oder integrierten Steuergeräts erfolgen. Die ersten, zweiten, dritten, vierten und fünften Lichtquellen können dabei als einfarbige oder mehrfarbige LEDs, z.B. RGB, ausgebildet sein.

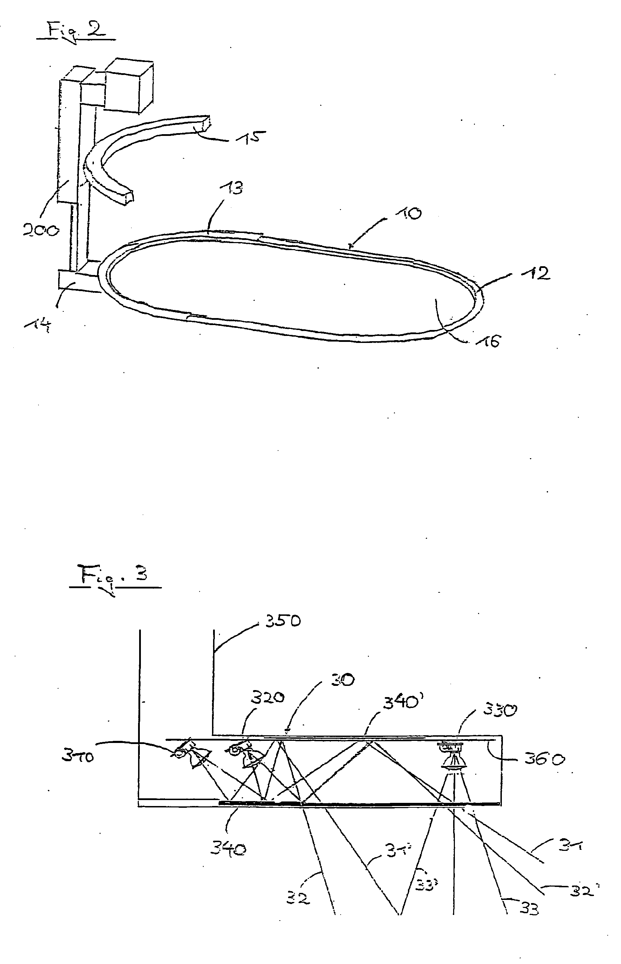

Figur 2 zeigt eine Prinzipskizze der Beleuchtung der Darstellungsfläche und deren Anordnung auf einer Befestigungseinheit. Die Darstellungseinheit 10 weist eine transluzente Darstellungsfläche 16 und eine erste Aufnahmeeinheit 12 auf, in der die Darstellungsfläche 16 angeordnet ist. Die transluzente Darstellungsfläche 16 ist dabei beispielsweise aus einem Kunststoffmaterial, wie z. B. Polyester, und mit einer Beschichtung, wie z. B. Polyurethan versehen. Daher weist die Darstellungsfläche 16 eine geringe Reflexionseigenschaft und gute Lichtstreuungseigenschaften auf, was bei einer Beleuchtung durch eine Lichtquelle eine gleichmäßige Ausleuchtung der Darstellungsfläche 16 bewirkt. Gleichzeitig ist die Darstellungsfläche 16 auch geeignet, die beispielsweise von einem Beamer ausgesandten Lichtstrahlen per Rückprojektion in das Blickfeld des liegenden Patienten zu bringen.

Ferner ist die erste Aufnahmeeinheit 12, die z.B. als Aluminiumrohr ausgebildet sein kann, in eine zweite Aufnahmeeinheit 13 einsetzbar. Die Befestigung der ersten Aufnahmeeinheit 12 in der zweiten Aufnahmeeinheit 13 erfolgt beispielsweise mittels Rastelementen, so dass eine einfache Austauschbarkeit möglich ist. Auch ist die zweite Aufnahmeeinheit 13 mit einem auf der Befestigungseinheit 200 angeordneten Haltebügel 14 gekoppelt. Der Haltebügel 14 ist vertikal teleskopartig aus der Befestigungseinheit ausziehbar, damit die Darstellungseinheit 10 ergonomisch demontiert und montiert werden kann.

Ferner ist auf der Befestigungseinheit 200 eine u-förmige Aufnahmeeinheit 15 angeordnet. Innerhalb dieser u-förmigen Aufnahmeeinheit 15 ist die erste Lichteinheit angeordnet, die mindestens eine erste Lichtquelle aufweist. Die erste Lichtquelle ist dabei derart in der Aufnahmeeinheit angeordnet und/ oder mit optischen Mitteln versehen, dass bei einer Beleuchtung der Darstellungsfläche 16, die von der ersten Lichtquelle ausgesandten Lichtstrahlen 11, 11' mit der Darstellungsfläche 16 einen spitzen Winkel, vorzugsweise 10°, ausbilden.

Figur 3 zeigt eine schematische Ansicht der Patientenbeleuchtung. Eine dritte Lichteinheit 30 ist dabei in einem Gehäuse 350 angeordnet. Die dritte Lichteinheit 30 weist in diesem Ausführungsbeispiel drei fünfte Lichtquellen 310, 320, 330 auf, wobei die Anzahl der fünften Lichtquellen nicht auf diese drei fünften Lichtquellen beschränkt sein muss.

In diesem Ausführungsbeispiel werden die fünften Lichtquellen 310, 320, 330 zur Beleuchtung eines in der Figur 3 nicht dargestellten Patientenbetts eingesetzt. Die fünften Lichtquellen 310, 320, 330 sind dabei durch einen Patienten oder durch ein externes, oder integriertes Steuergerät einzeln ansteuerbar. Somit lassen sich beispielsweise einzelne Bereiche des Patientenbetts separat beleuchten. Die Lichtstrahlen 31, 31', 32, 32' der jeweiligen fünfte Lichtquelle 310, 320 werden ausgehend von der fünften Lichtquelle jeweils durch ein im Gehäuse 350 angeordnetes Reflektormittel 340, 340' zweimal umgelenkt, um den Patienten nicht zu blenden.

Dabei wird durch die erste fünfte Lichtquelle 310 der Fußbereich, durch die zweite fünfte Lichtquelle 320 der Mittelbereich und durch die dritte fünfte Lichtquelle 330 der Kopfbereich des Patienten beleuchtet.

Die Erfindung betrifft eine Beleuchtungsvorrichtung für eine Medizinische Versorgungseinheit. Die Beleuchtungsvorrichtung weist mehrere auf mehreren Befestigungseinheiten angeordnete Lichteinheiten auf, die zur Beleuchtung eines Raums und einer Darstellungsfläche dienen. Dabei können beispielsweise die Farbtemperatur und deren Verteilung, und die Helligkeit der einzelnen Lichteinheiten aufeinander abgestimmt sein.

## Patentansprüche

1. Medizinische Versorgungseinheit mit einer Beleuchtungsvorrichtung, aufweisend mindestens eine erste Lichteinheit, die an einer Befestigungseinheit (200) angeordnet ist, und eine auf der Befestigungseinheit (200) angeordnete transluzente Darstellungsfläche (16), auf die mindestens eine, innerhalb der ersten Lichteinheit angeordnete, erste Lichtquelle gerichtet ist, wobei die transluzente Darstellungsfläche (16) auf der Befestigungseinheit (200) derart angeordnet ist und eine derartige Größe aufweist, dass eine Beleuchtung der transluzenten Darstellungsfläche (16) und/ oder eine Bildprojektion auf der transluzenten Darstellungsfläche (16) möglich ist, **dadurch gekennzeichnet, dass** die Darstellungsfläche (16) innerhalb einer ersten Aufnahmeeinheit (12) angeordnet ist, die erste Aufnahmeeinheit (12) in einer auf der Befestigungseinheit (200) angeordneten zweiten Aufnahmeeinheit (13) einsetzbar ist, und die zweite Aufnahmeeinheit (13) in einer Richtung senkrecht zur Darstellungsfläche (16) verschiebbar ist.

2. Medizinische Versorgungseinheit nach Anspruch 1, wobei die Darstellungsfläche (16) aus einem Kunststoffgewebe besteht.

3. Medizinische Versorgungseinheit nach einem der vorhergehenden Ansprüche, wobei die auf die Darstellungsfläche (16) gerichtete erste Lichtquelle in einer Aufnahmeeinheit (15) angeordnet ist, wobei sich die Aufnahmeeinheit (15) oberhalb der zu einer Raumdecke (100) gerichteten Oberfläche der Darstellungsfläche (16) befindet.

4. Medizinische Versorgungseinheit nach Anspruch 3, wobei die Aufnahmeeinheit (15) u-förmig ist.

5. Medizinische Versorgungseinheit nach einem der vorhergehenden Ansprüche, wobei die von der zur Darstellungsfläche (16) gerichteten ersten Lichtquelle ausgesandten Lichtstrahlen (11, 11') mit der Darstellungsfläche (16) einen spitzen Winkel ausbilden.

6. Medizinische Versorgungseinheit nach einem der vorhergehenden Ansprüche, wobei mindestens eine, auf einer Befestigungseinheit (200, 200'. 200'') angeordnete, zweite Lichteinheit mindestens eine zweite Lichtquelle (20), die auf die Raumdecke (100) gerichtet ist und/ oder eine dritte Lichtquelle (22), die auf die Raumseitenwände (101) gerichtet ist, und/ oder eine vierte Lichtquelle (24), die auf den Raumboden (103) gerichtet ist, aufweist.

7. Medizinische Versorgungseinheit nach Anspruch 6, wobei der jeweilige von der zweiten und/ oder dritten und/ oder vierten Lichtquelle (20, 22, 24) ausgesandte Lichtstrahl auf die Raumdecke (100), den Raumboden (103) und die Raumseitenwände (101) mit der Raumdecke (100), dem Raumboden (103) und den Raumseitenwänden (101) einen spitzen Winkel einschließt.

8. Medizinische Versorgungseinheit nach Anspruch 5 bis 7, wobei der spitze Winkel einen Wert von 10° aufweist.

9. Medizinische Versorgungseinheit nach einem der vorhergehenden Ansprüche, wobei die Beleuchtungsvorrichtung eine Schnittstelle für eine Ansteuerung aufweist.

10. Medizinische Versorgungseinheit nach einem der vorhergehenden Ansprüche, wobei die Bildprojektion auf die Darstellungsfläche (16) mittels eines Beamers (40) erfolgt.

11. Medizinische Versorgungseinheit nach einem der vorhergehenden Ansprüche, wobei die Beleuchtungsvorrichtung eine Kamera und/ oder einen Sensor, insbesondere einen Lichtsensor, aufweist.

12. Medizinische Versorgungseinheit nach einem der vorhergehenden Ansprüche, wobei mindestens eine fünfte Lichtquelle (310, 320, 330) in einer dritten Lichteinheit unterhalb der zum Raumboden (103) gerichteten Oberfläche der Darstellungsfläche (16) angeordnet ist, wobei die dritte Lichteinheit an der Befestigungseinheit (200) befestigbar ist.

13. Medizinische Versorgungseinheit nach Anspruch 12, wobei jede fünfte Lichtquelle (310, 320, 330) einzeln ansteuerbar ist.

## Claims

1. Medical supply unit with a lighting device, comprising at least one first light unit arranged on a fixing unit (200) and a translucent display area (16) which is arranged on the fixing unit (200) and to which at least one light source arranged within the first light unit is directed, wherein the translucent display area (16) is arranged on the fixing unit (200) and has a size such that illumination of the translucent display area (16) and/or image projection on the translucent display area (16) are enabled,
**characterized in that**
the display area (16) is arranged within a first receiving unit (12),
the first receiving unit (12) is insertable into a second receiving unit (13) arranged on the fixing unit (200) and the second receiving unit (13) is slidable in a direction perpendicular to the display area (16).

2. Medical supply unit according to claim 1, wherein the display area (16) is made of synthetic fabric.

3. Medical supply unit according to any one of the preceding claims, wherein the first light source directed to the display area (16) is arranged in a receiving unit (15), wherein the receiving unit (15) is located above a surface of the display area (16) facing a room ceiling (100).

4. Medical supply unit according to claim 3, wherein the receiving unit (15) is U-shaped.

5. Medical supply unit according to any one of the preceding claims, wherein the light beams (11, 11') emitted from the first light source directed to the display area (16) enclose an acute angle with the display area (16).

6. Medical supply unit according to any one of the preceding claims, wherein at least one second light unit arranged on a fixing unit (200, 200', 200") comprises at least one second light source (20) directed to the room ceiling (100) and/or one third light source (22) directed to the room sidewalls (101) and/or one fourth light source (24) directed to the room floor (103).

7. Medical supply unit according to claim 6, wherein the respective light beams emitted from the second and/or third and/or fourth light sources (20, 22, 24) to the room ceiling (100), the room floor (103) and the room sidewalls (101) enclose an acute angle with the room ceiling (100), the room floor (103) and the room sidewalls (101).

8. Medical supply unit according to claim 5 to 7, wherein the acute angle has a value of 10°.

9. Medical supply unit according to any one of the preceding claims, wherein the illumination device comprises a control interface.

10. Medical supply unit according to any one of the preceding claims, wherein the image projection on the display area (16) is performed by a beamer (40).

11. Medical supply unit according to any one of the preceding claims, wherein the illumination device comprises a camera and/or a sensor, in particular a light sensor.

12. Medical supply unit according to any one of the preceding claims, wherein at least one fifth light source (310, 320, 330) is arranged in a third light unit below the surface of the display area (16) facing the room floor (103), wherein the third light unit is fixable on the fixing unit (200).

13. Medical supply unit according to claim 12, wherein each fifth light source (310, 320, 330) is individually controllable.

## Revendications

1. Unité de soins médicaux pourvue d'un dispositif d'éclairage, comportant au moins une première unité lumineuse disposée sur une unité de fixation (200) et une surface de projection (16) translucide disposée sur l'unité de fixation (200) et vers laquelle est dirigée au moins une première source lumineuse disposée à l'intérieur de la première unité lumineuse, la surface de projection (16) translucide présentant une disposition sur l'unité de fixation (200) et une grandeur permettant l'éclairage de la surface de projection (16) translucide et/ou une projection d'image sur la surface de projection (16) translucide, **caractérisée en ce que** la surface de projection (16) est disposée à l'intérieur d'une première unité d'enregistrement (12), **en ce que** ladite première unité d'enregistrement (12) peut être mise en place dans une deuxième unité d'enregistrement (13) disposée sur l'unité de fixation (200), et **en ce que** ladite deuxième unité d'enregistrement (13) est déplaçable dans une direction perpendiculaire à la surface de projection (16).

2. Unité de soins médicaux selon la revendication 1, dans laquelle la surface de projection (16) est en tissu synthétique.

3. Unité de soins médicaux selon l'une des revendications précédentes, dans laquelle la première source lumineuse dirigée vers la surface de projection (16) est disposée dans une unité d'enregistrement (15), ladite unité d'enregistrement (15) se trouvant au-dessus de la face opposée au plafond (100) de la surface de projection (16).

4. Unité de soins médicaux selon la revendication 3, dans laquelle l'unité d'enregistrement (15) est en forme de U.

5. Unité de soins médicaux selon l'une des revendications précédentes, dans laquelle les rayons lumineux (11, 11') émis par la première source lumineuse dirigée vers la surface de projection (16) forment un angle aigu avec la surface de projection (16).

6. Unité de soins médicaux selon l'une des revendications précédentes, dans laquelle au moins une deuxième unité lumineuse disposée sur une unité de fixation (200, 200', 200") comporte au moins une deuxième source lumineuse (20) dirigée vers le plafond (100) et/ou une troisième source lumineuse (22) dirigée vers les murs latéraux (101) et/ou une quatrième source lumineuse (24) dirigée vers le plancher (103).

7. Unité de soins médicaux selon la revendication 6, dans laquelle le rayon lumineux respectivement émis par la deuxième et/ou la troisième et/ou la quatrième source lumineuse (20, 22, 24) vers le plafond (100), le plancher (103) et les murs latéraux (101), forme un angle aigu avec le plafond (100), le plancher (103) et les murs latéraux (101).

8. Unité de soins médicaux selon les revendications 5 à 7, dans laquelle l'angle aigu a une valeur de 10°.

9. Unité de soins médicaux selon l'une des revendications précédentes, dans laquelle l'unité de soins médicaux comporte une interface pour une commande.

10. Unité de soins médicaux selon l'une des revendications précédentes, dans laquelle l'image est projetée par un projecteur (40) sur la surface de projection (16).

11. Unité de soins médicaux selon l'une des revendications précédentes, dans laquelle le dispositif d'éclairage comporte une caméra et/ou un capteur, en particulier un capteur de lumière.

12. Unité de soins médicaux selon l'une des revendications précédentes, dans laquelle au moins une cinquième source lumineuse (310, 320, 330) est disposée dans une troisième unité lumineuse en dessous de la face opposée au plancher (103) de la surface de projection (16), la troisième unité lumineuse pouvant être fixée à l'unité de fixation (200).

13. Unité de soins médicaux selon la revendication 12, dans laquelle chaque cinquième source lumineuse (310, 320, 330) peut être commandée individuellement.
